# NEUE EUROPÄISCHE PATENTSCHRIFT

(11) **EP 1 019 718 B2**
(45) Veröffentlichungstag und Bekanntmachung des Hinweises auf die Entscheidung über den Einspruch: **21.03.2007**
(45) Hinweis auf die Patenterteilung: 18.12.2002
(21) Anmeldenummer: 98951507.7
(22) Anmeldetag: 09.10.1998
(51) Int. Cl.: G01N 33/487, C12M 1/34

(54) **ZUR ZELLUNTERSUCHUNG MITTELS DER PATCH CLAMP-METHODE BESTIMMTE VORRICHTUNG UND VERFAHREN**
DEVICE AND METHOD FOR EXAMINING CELLS USING THE PATCH-CLAMP METHOD
DISPOSITIF ET PROCEDE POUR EXAMINER DES CELLULES A L'AIDE DE LA METHODE PATCH-CLAMP

(30) Priorität: 09.10.1997 DE 19744649
(43) Veröffentlichungstag der Anmeldung: 19.07.2000
(73) Patentinhaber: Fraunhofer-Gesellschaft zur Förderung der angewandten Forschung e.V., 80686 München (DE)
(72) Erfinder: MEYER, Jörg-Uwe, D-66386 St. Ingbert (DE)
(74) Vertreter: Rösler, Uwe
(86) Internationale Anmeldenummer: PCT/EP1998/006418
(87) Internationale Veröffentlichungsnummer: WO 1999/019729

(56) Entgegenhaltungen:
- EP-A- 0 542 422
- WO-A-96/13721
- DE-A- 3 342 504
- DE-A- 3 805 808
- US-A- 5 092 184
- US-A- 5 183 744
- US-A- 5 643 742
- Fischmeister & et al. (1986), Eur.J. Physiol., vol. 406, pp.73-82

## Beschreibung

### Technisches Gebiet

Die Erfindung bezieht sich auf eine zur Zelluntersuchung mittels der Patch Cfamp-Methode bestimmte Vorrichtung sowie auf ein dazu bestimmtes Verfahren. Weiterhin ist Gegenstand der Erfindung die Verwendung einer an sich bekannten bekannten Vorrichtung zur Messung bioelektrischer Signale zur Zelluntersuchung.

### Stand der Technik

Die Patch Clamp-Methode ist ein Verfahren, um bioelektrische Signale, namentlich das elektrische Potential im Innern einer Zelle und den durch ionische Transportprozesse, die sogenannte lonenkanäle zustandekommenden Strom durch eine Zellmembran zu messen.

Mit diesem Verfahren ist es möglich, den Strom durch eine Zellmembran sogar separat für einen spezifischen lonenkanal zu messen und dadurch Erkenntnisse über die Funktionsweise der Zellmembran zu gewinnen. Messungen mithilfe der Patch Clamp-Methode werden in der Neurowissenschaft vorgenommen, um die Wirkung von Pharmaka und Chemotherapeutika auf die Aktivität von lonenkanälen zu erforschen.

Vorrichtungen zur Messung bioelektrischer Signale sind aus der DE 38 05 808 A1 sowie den US-Druckschriften US 4599315, US 5229163 und US 5643742 bekannt.

Aus der Patch Clamp-Methode wurden verschiedene Varianten entwickelt, um die elektrische Leitfähigkeit in einzelnen Membranabschnitten zu bestimmen. Für die Untersuchung wird die Membran einer Zelle, üblicherweise einer Nervenzelle, mit einer als Mikropipette dienenden Glaskapillare angesaugt und je nach Variante auch durchstoßen, um das intrazelluläre Potential von typischerweise 1 - 500 µV mit einer in der Glaskapillare befindlichen Elektrode gegenüber einer Referenzelektrode zu messen. Dabei ist die Zelle mechanisch fest mit der Mikropipette verbunden und steht über ihre Membran mit der umgebenden Lösung in Kontakt.

Gemäß einer anderen Variante wird ein angesaugter Teil einer Zellmembran von der Zelle abgetrennt, um die Leitfähigkeit dieses Membranstücks (patch) zu ermitteln. Bezüglich der Art der Membranabtrennung sind verschiedene Varianten geläufig, um das Membranstück für die nachfolgenden Messungen in beiderlei Orientierungen (inside-out patch bzw. outside-out patch) an der Mikropipette zu befestigen.

Die Messung des Potentials innerhalb der Zelle bzw. die Messung von Strömen durch die Zellmembran hindurch erfolgt mit Hilfe von Mikroelektroden, die sich innerhalb der Mikropipette befinden können oder mit bekannten Verfahren wie der Dünnschichttechnik auf Substraten hergestellt werden, auf denen die zu untersuchenden Neuronen positioniert werden müssen.

Obwohl die Patch Clamp-Methode sich seit ihrer Einführung in der Neurowissenschaft zu einer Routinemessung in neurobiologischen Labors entwickelt hat, ist sie meßtechnisch äußerst anspruchsvoll und erfordert bislang noch eine mikroskopische Kontrolle des Eingriffs an der zu untersuchenden Zelle. Nachteilig ist dabei vor allem, daß dieses Verfahren für den Umgang mit der einzelnen Nervenzelle ein erhebliches Geschick und Feingefühl seitens des Operateurs voraussetzt; so müssen die Neuronen beispielsweise auf einem Substrat aufgesucht werden und gegebenenfalls auch unmittelbar an Mikroelektroden an der Substratoberfläche positioniert werden, um die erforderlichen Messungen durchführen zu können.

Infolge des manuellen Umgangs mit der einzelnen Zelle ist die Patch Clamp-Methode bislang mit einem erheblichen Zeitaufwand verbunden. Insbesondere bei der Untersuchung einer Vielzahl von Zellen ist dieser Nachteil um so gravierender, da die Zellen nach ihrer lsolierung vom Gewebe räumlich beliebig verteilt sind und aus diesem Grunde nur nacheinander aufgesucht, plaziert und untersucht werden können.

Dennoch wird in der WO 96/13721 vorgeschlagen die Durchführung der Patch Clamp-Methode zumindest teilweise zu automatisieren, indem die Zuführung der zu untersuchenden Zellen zum Meßort unter Zuhilfenahme eines Autosamplers erfolgt. Hierbei wird mittels eines Düsensystems automatisch eine Zellprobe aus einer Küvettenanordnung entnommen und über entsprechende Schlauchleitungen zum Meßort verbracht. Die Messung selbst erfolgt wieder auf konventionellem Wege, indem ein Operatuer mithilfe der Patch Pipette die eigentliche Messung durchführt. Eine gleichzeitige Untersuchung von einer Vielzahl von Zellproben ist mit dieser bekannten Anordnung jedoch nicht möglich.

### Darstellung der Erfindung

Der Erfindung liegt daher die Aufgabe zugrunde, eine Vorrichtung sowie ein Verfahren anzugeben, mit denen mit der Patch Clamp-Methode eine Vielzahl von Zellen gleichzeitig zu untersucht, die Untersuchung der Zellen zu automatisiert und so auf die bislang erforderliche mikroskopische Kontrolle verzichtet werden kann. Ferner soll die Untersuchung einer Vielzahl von Zellen auf kleinstem Raum ermöglicht und schließlich der für die Patch Clamp-Messung nötige Zeitaufwand drastisch minimiert werden.

Eine erfindungsgemäße Lösung dieser Aufgabe ist für die Vorrichtung im Anspruch 1 und für das Verfahren im Anspruch 6 angegeben. Weiterbildungen der Erfindung sind Gegenstand der abhängigen Ansprüche.

Die Aufgabe wird erfindungsgemäß gelöst durch eine Vorrichtung mit einer flächigen Anordnung einer ersten Anzahl von Mikroküvetten zur Aufnahme von Zellen und mit einer flächigen Anordnung einer zweiten Anzahl von Mikropipetten mit Messelektroden, die relativ zur flächigen Anordnung der Mikroküvetten positionierbar ist, um eine Vielzahl in den Mikroküvetten befindlicher Zellen gleichzeitig zu untersuchen. Die Messung der bioelektrischen Signale erfolgt dabei mittels der Patch Clamp-Methode.

Die Aufgabe wird erfindungsgemäß ferner durch ein Verfahren gelöst, wonach eine Vielzahl von Zellen angeordnet wird und mittels einer Vielzahl von Mikropipetten eine Vielzahl angeordneter Zellen gleichzeitig untersucht wird.

Durch die systematische räumliche Anordnung sowohl der zur Aufnahme der Zellen bestimmten Mikroküvetten als auch der Mikropipetten wird es erstmals möglich, die Patch Clamp-Methode zu automatisieren und auf diese Weise zeit- und kostensparend durchzuführen. Die zu untersuchenden Neuronen werden von einer flächigen Anordnung von Mikroküvetten - in der Regel ein Substrat mit gleichmäßig verteilten Vertiefungen in seiner Oberfläche - aufgenommen und so auf exakt bestimmbaren Positionen gehalten. Eine flächige Anordnung von Mikropipetten ermöglicht bei genauer Positionierung gegenüber der Anordnung der in den Mikroküvetten befindlichen Zellen die gleichzeitige Durchführung einer Vielzahl von Einzelmessungen, die bislang nur nacheinander und bei gleichzeitiger Beobachtung durch ein Mikroskop durchgeführt werden konnten.

Die Mikropipetten bzw. Mikroküvetten können auf den entsprechenden Flächen auf engstem Raum angeordnet werden und ermöglichen so den Bau sehr kompakter Meßvorrichtungen. Die Küvetten und Pipetten werden auf den dafür vorgesehenen Flächen vorzugsweise regelmäßig verteilt, d.h. sie bilden bei festem gegenseitigem Abstand untereinander ein gleichmäßiges Raster. Die genaue Gestalt der Raster wird sich in der Praxis an Größe und Form der Mikropipetten bzw. der Mikroküvetten orientieren.

Eine erfindungsgemäß bevorzugte Ausführungsform der Vorrichtung sieht vor, daß die flächige Anordnung von Mikroküvetten ein der flächigen Anordnung von Mikropipetten angepaßtes, aber in mindestens einer Richtung kleineres Rastermaß besitzt und daß die Anordnung von Mikroküvetten und die Anordnung von Mikropipetten relativ zueinander seitlich versetzbar sind, um wiederholt eine Vielzahl von Zellen gleichzeitig zu untersuchen.

Im einfachsten Fall einer regelmäßigen Anordnung entspricht der Abstand benachbarter Mikroküvetten einem ganzzahligen Bruchteil des Abstandes benachbarter, gegenüber den Mikroküvetten größerer Mikropipetten, wodurch eine wesentlich erhöhte Zahl von Zellen gleichzeitig aufgenommen werden kann als bei einer Anordnung gleich vieler Küvetten wie Pipetten. Die Anzahl gleichzeitiger Messungen ist nur noch durch die Größe der Mikropipetten bzw. durch die für sie vorgesehene Flächengröße begrenzt. Zur Untersuchung sämtlicher in den Mikroküvetten befindlicher Zellen sieht die entsprechende Ausführungsform des Verfahrens vor, daß die Vielzahl von Mikropipetten relativ zu einer Anordnung von Zellen versetzt wird und wiederholt eine Vielzahl angeordneter Zellen untersucht wird. Auf diese Weise kann die erfindungsgemäße Vielfachmessung in kurzen Zeitabständen wiederholt werden, ohne daß es eines zwischenzeitlichen Austausches der Zellen bedarf. Dies führt zu einer weiteren Steigerung der Durchsatzzahlen.

Eine weitere Ausführungsform der Erfindung sieht einen Mikromotor zum Positionieren und/oder zum Versetzen der Anordnung von Mikroküvetten und der Anordnung von Mikropipetten relativ zueinander vor. Der Mikromotor zum Absenken der Mikropipettenanordnung auf die Mikroküvettenanordnung und zum seitlichen Verfahren beider gegeneinander gewährleistet mithilfe der heutigen Feinmechanik und Mikrotechnologie den punktgenauen Kontakt von Neuronen und Pipetten, wodurch die vorgestellte Erfindung die erstmalige Untersuchung einer Vielzahl von Zellen ermöglicht. Dabei kann die Pipettenanordnung oder auch die Küvettenanordnung durch den Mikromotor verfahren werden.

Eine weitere Ausführungsform der Erfindung sieht eine Zellzuführeinrichtung und/oder eine Spüleinrichtung zum Entfernen von Zellen vor. Dies führt zu einer weiteren Automatisierung der Messungen und damit zu einer weiteren Zeitersparnis insbesondere im Falle einer Vielfachmessung, die die Aufnahmekapazität der Küvettenansammlung übersteigt.

Eine weitere Ausführungsform der Erfindung sieht einen Filter auf der Rückseite der Anordnung der Mikroküvetten vor. Da die zu untersuchenden Zellen von der der Pipettenanordnung zugewandten Seite aus in die Mikroküvetten eingebracht werden und die Mikroküvetten vorzugsweise in Richtung der Pipetten konisch aufgeweitet sein können, um die Zellen sicher aufzunehmen, empfiehlt sich eine Spülung der Küvettenanordnung von der Rückseite her. In diesem Fall ist ein Filter vorteilhaft, um ein Verstopfen der Mikroküvetten durch kleine Partikel zu verhindern.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Figur 1: einen Querschnitt durch eine erfindungsgemäßen Vorrichtung während der Patch Clamp-Messung,
- Figur 2: eine vergrößerte Detailansicht aus Figur 1,
- Figur 3: einen Querschnitt einer erfindungsgemäßen Ausführungsform der Vorrichtung, und
- Figur 4: eine perspektivische Ansicht der erfindungsgemäßen Anordnungen von Mikroküvetten und Mikropipetten.

### Wege zur Ausführung der Erfindung, gewerbliche Verwendbarkeit

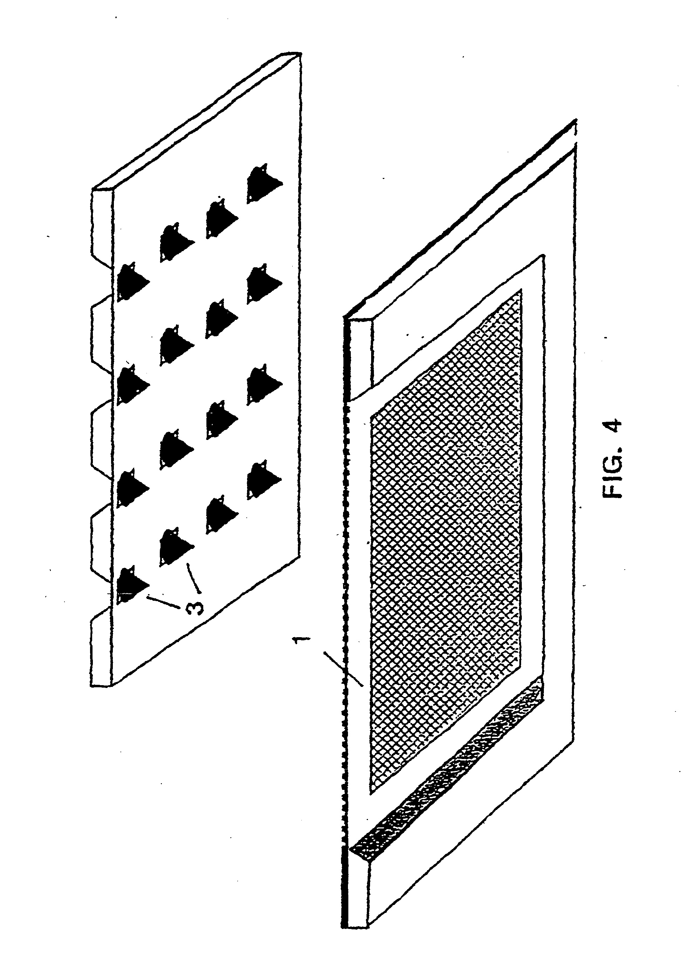

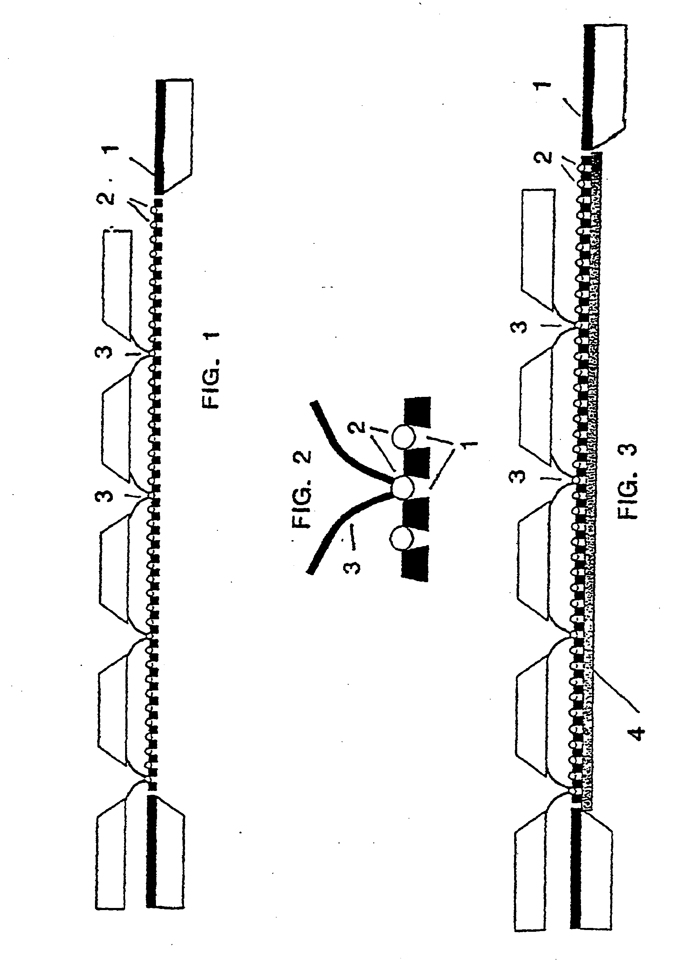

Die Figuren 1 und 2 zeigen schematisch eine erfindungsgemäße Vorrichtung während der Patch Clamp-Messung.

Eine Mikroküvettenanordnung 1 weist eine Vielzahl im Raster angeordneter Mikroküvetten zur Aufnahme einer Vielzahl von Zellen 2 auf. Einige der Zellen werden von einer Mikropipettenanordnung 3 berührt bzw. von den Mikropipetten angesaugt. Mit dieser Anordnung von n x n Pipetten können n² Messungen gleichzeitig durchgeführt werden.

Figur 3 zeigt eine Ausführungsform der erfindungsgemäßen Vorrichtung mit einem unterhalb der Mikroküvetten befindlichen Filter 4, gleicht ansonsten der Figur 1.

Die räumliche Anordnung der Mikroküvettenanordnung (MKA) und der Mikropipettenanordnung (MPA) ist in Figur 4 dargestellt. Die MPA befindet sich über der MKA, wobei die spitz zulaufenden Öffnungen der Mikropipetten der MKA zugewandt sind. Die MPA kann durch einen nicht abgebildeten Mikromotor relativ zur MKA verfahren werden.

Für eine ungefähre Einschätzung der typischen Abmessungen der erfindungsgemäßen Vorrichtung werden nachstehend ohne jegliche Beschränkung der Allgemeinheit einige exemplarische Zahlenangaben genannt:

Auf der Mikroküvettenanordnung von der Größe eines Quadratzentimeters befinden sich auf einer Fläche von 8 x 8 mm2 entlang jeder Richtung des quadratischen Rasters 160 Küvetten und somit insgesamt 25600 Küvetten im gegenseitigen Abstand von 50 µm. Die Mikropipettenanordnung weist auf einer Fläche von mindestens 2 x 2 mm² 16 im quadratischen Raster angeordnete Mikropipetten (Nozzles) mit einem gegenseitigen Abstand von 400 µm auf. Das Rastermaß der Mikropipettenanordnung entspricht hier also dem achtfachen Rastermaß der Mikroküvettenanordnung. Um alle in der MKA befindlichen Zellen zu untersuchen, kann die MPA in beiden Richtungen parallel zum MKA in 40 Schritten verfahren und damit in insgesamt 1600 unterschiedliche Positionen gebracht werden. Mikroküvettenanordnung sowie Mikropipettenanordnung werden mithilfe lithographischer Methoden der Halbleitertechnik, die Elektroden der MPA in Dünnfilmtechnik gefertigt.

Mikromechanische Strukturen dienen zur Justierung des Motors; die Komponenten werden feinmechanisch und mikrotechnisch verbunden.

Ein Mikroprozessor steuert die Mikroaktoren und erfaßt die anfallenden Meßdaten, die dann elektronisch ausgewertet werden.

Mit der erfindungsgemäßen Vorrichtung und dem beschriebenen Verfahren können derzeit bereits einige Tausend Messungen in wenigen Minuten durchgeführt werden.

## Patentansprüche

1. Vorrichtung zur Erfassung des elektrischen Potentials im Inneren einer biologischen Zelle sowie des elektrischen Stromes, der bei ionischen Transportprozessen durch Zellwände auftritt,
**dadurch gekennzeichnet, dass** eine flächige Anordnung einer ersten Anzahl von Mikroküvetten (1) vorgesehen ist,
dass eine flächige Anordnung einer zweiten Anzahl von Mikropipetten (3) vorgesehen ist, die zur elektrischen Potential und Stromerfassung Mikroelektroden aufweisen, und
dass die flächige Anordnung von Mikropipetten (3) relativ zur flächigen Anordnung der Mikroküvetten derart positionierbar ist, daß die Mikropipetten mit, in Mikroküvetten eingebrachter Zellen kontaktierbar sind und eine gleichzeitige Untersuchung einer Vielzahl von Zellen erfolgt.

2. Vorrichtung nach Anspruch 1,
**dadurch gekennzeichnet, daß** die flächige Anordnung von Mikroküvetten ein der flächigen Anordnung von Mikropipetten angepaßtes, aber in mindestens einer Richtung kleineres Rastermaß besitzt und daß die Anordnung von Mikroküvetten und die Anordnung von Mikropipetten relativ zueinander seitlich versetzbar sind, um wiederholt eine Vielzahl von Zellen gleichzeitig zu untersuchen.

3. Vorrichtung nach Anspruch 1 oder 2,
**gekennzeichnet durch** einen Mikromotor zum Positionieren und/oder zum Versetzen der Anordnung von Mikroküvetten und der Anordnung von Mikropipetten relativ zueinander.

4. Vorrichtung nach einem der Ansprüche 1 bis 3,
**gekennzeichnet durch** eine Zellzuführeinrichtung und/oder eine Spüleinrichtung zum Entfernen von Zellen.

5. Vorrichtung nach einem der Ansprüche 1 bis 4,
**gekennzeichnet durch** einen Filter (4) auf der Rückseite der Anordnung der Mikroküvetten.

6. Verfahren zur Zelluntersuchung mittels der Patch-Clamp-Methode **dadurch gekennzeichnet, dass** eine Vielzahl von Zellen (2) in einer flächigen Anordnung von Mikroküvetten angeordnet wird und mit Hilfe einer Vielzahl von Mikropipetten (3) mit Mikroelektroden eine Vielzahl angeordneter Zellen gleichzeitig untersucht wird.

7. Verfahren nach Anspruch 6,
**dadurch gekennzeichnet, daß** die Vielzahl von Mikropipetten relativ zu einer Anordnung von Zellen versetzt wird und wiederholt eine Vielzahl angeordneter Zellen untersucht wird.

8. Verwendung einer Vorrichtung zur Zelluntersuchung mittels der Patch Clamp-Methode, die eine flächige Anordnung einer ersten Anzahl von Mikroküvetten (1) zur Aufnahme von Zellen (2) und
eine flächige Anordnung einer zweiten Anzahl von Mikropipetten (3) mit Mikroelektroden aufweist, die relativ zur flächigen Anordnung der Mikroküvetten derart positionierbar ist, daß eine Vielzahl von in den Mikroküvetten befindlicher Zellen gleichzeitig untersuchbar ist.

## Claims

1. A device for determining the electric potential inside a biological cell and the electric current occurring in ionic transport processes through cell walls,
**wherein** a plane arrangement of a first number of micro-cuvettes (1) is provided,
a plane arrangement of a second number of micro-pipettes (3) is provided, which are provided with micro-electrodes for determining said electric portential and said electric current, and
said plane arrangement of micro-pipettes (3) is positionable in relation to said plane arrangement of micro-cuvettes in such a manner that said micro-pipettes are contactable with cells conveyed into said micro-cuvettes and examination of a multiplicity of cells occurs simultaneously.

2. Device according to claim 1,
**wherein** said plane arrangement of micro-cuvettes has grid dimensions matching said plane arrangement of micro-pipettes but at least smaller in one direction and by said arrangement of micro-cuvettes and said arrangement of pipettes being laterally offset in relation to each other in order to repeatedly examine a multiplicity of cells simultaneously.

3. Device according to claim 1 or 2,
comprising further a micro-motor for positioning and/or offsetting said arrangement of micro-cuvettes and said arrangement of micro-pipettes in relation to each other.

4. Device according to one of the claims 1 to 3,
comprising further a cell feed device and /or a rinsing device for removing said cells being provided.

5. Device according to one of the claims 1 to 4,
comprising further a filter (4) on the rear side of said arrangement of micro-cuvettes being provided.

6. A process provided for examining cells using the patch-clamp method, wherein a multiplicity of cells (2) are disposed in a two-dimensional arrangement of micro-cuvettes and a multiplicity of disposed cells are examined simultaneously with the aid of multiplicity of micro-pipettes (3) which are provided with micro-electrodes.

7. The process according to claim 6,
**wherein** said multiplicity of micro-pipettes and an arrangement of cells are offset in relation to each other and a multiplicity of disposed cells is repeatedly examined.

8. Use of a device for examining cells using said patch-clamp method, having a plane arrangement of a first number of micro-cuvettes (1) for receiving cells (2) and
a plane arrangement of a second number of micro-pipettes (3), which are provided with micro-electrodes and which can be positioned in relation to said plane in such a manner that a multiplicity of cells located in said micro-cuvettes can be examined simultaneously.

## Revendications

1. Appareil à détecter le potentiel électrique à l'intérieur d'une cellule biologique ainsi que le courant électrique, qui est présent au cours des processus de transfert ionique à travers des membranes de cellules,
**caractérisé en ce qu'**un arrangement bidimensionnel d'un premier nombre de microcuvettes (1) est disposé,
**en ce qu'**un arrangement bidimensionnel d'un deuxième nombre de micropipettes (3) est disposé, qui comprennent des micro électrodes à détecter le potentiel électrique et à détecter le courant, et
**en ce que** l'arrangement bidimensionnel de micropipettes (3), relatif à l'arrangement bidimensionnel des microcuvettes, est apte à être positionné de manière, qu'on puisse contacter les micropipettes avec les cellules introduites dans des microcuvettes et qu'on achève une analyse simultanée d'une pluralité de cellules.

2. Appareil selon la revendication 1,
**caractérisé en ce que** ledit arrangement bidimensionnel de microcuvettes présente une dimension de trame adaptée à l'arrangement bidimensionnel des micropipettes, mais plus petite en au moins un sens, et **en ce que** l'arrangement de microcuvettes et l'arrangement de micropipettes sont déplaçables latéralement, l'un relativement à l'autre, pour l'étude simultanée répétée d'une pluralité de cellules.

3. Appareil selon la revendication 1 ou 2,
**caractérisé par** un micromoteur à positionner et/ou déplacer l'arrangement des microcuvettes et l'arrangement des micropipettes, l'un relativement à l'autre.

4. Appareil selon une quelconque des revendications 1 à 3,
**caractérisé par** un moyen d'alimentation de cellules et/ou un moyen de rinçage à séparer des cellules.

5. Appareil selon une quelconque des revendications 1 à 4,
**caractérisé par** un filtre (4) du côté arrière de l'arrangement des microcuvettes.

6. Procédé d'analyse de cellules moyennant la méthode dite patch clamp, charactérisé en ce qu'une pluralité de cellules (2) est disposée dans une arrangement bi-dimensionelle de microcuvettes et en ce qu'une pluralité de cellules disposée est analysée, de façon simultanée, moyennant une pluralité de micropipettes (3) aux micro électrodes.

7. Procédé selon la revendication 6,
**caractérisé en ce que** ladite pluralité de micropipettes est déplacée relativement à un arrangement de cellules, et **en ce qu'**une pluralité de cellules disposées est analysée de manière répétitive.

8. Utilisation d'un appareil d'analyse de cellules par application de la méthode dite patch clamp, qui comprend un arrangement bidimensionnel d'un premier nombre de microcuvettes (1) à recevoir des cellules (2), et
un arrangement bidimensionnel d'un deuxième nombre de micropipettes (3) aux micro électrodes, qui se peut positionner relativement audit arrangement bidimensionnel des microcuvettes de façon, qu'on puisse analyser une pluralité de cellules en même temps, qui se trouvent dans lesdites microcuvettes.
